# EUROPEAN PATENT SPECIFICATION

(11) **EP 2 782 527 B1**
(45) Date of publication and mention of the grant of the patent: **18.11.2015**
(21) Application number: 12795034.3
(22) Date of filing: 21.11.2012
(51) Int. Cl.: A61F 2/46, A61F 2/34

(54) **ASSEMBLY COMPRISING A MEDICAL IMPLANT AND AN INSTRUMENT HEAD**
ANORDNUNG MIT EINEM MEDIZINISCHEN IMPLANTAT UND EINEM INSTRUMENTENKOPF
ENSEMBLE COMPORTANT UN IMPLANT MÉDICAL ET UNE TÊTE D'INSTRUMENT

(30) Priority: 23.11.2011 GB 201120194; 23.11.2011 GB 201120197
(43) Date of publication of application: 01.10.2014
(73) Proprietor: DePuy (Ireland), County Cork (IE)
(72) Inventor: TAYLOR, Andrew, Hampshire SO16 7NS (GB); BIRD, Timothy, Hampshire SO16 7NS (GB); HUNT, Toby, Hampshire SO16 7NS (GB)
(74) Representative: Alton, Andrew
(86) International application number: PCT/GB2012/052882
(87) International publication number: WO 2013/076484

(56) References cited:
- EP-A2- 0 916 324
- EP-A2- 1 634 552
- WO-A1-2010/146398
- US-A1- 2002 177 854

## Description

The present invention relates in general to the field of orthopaedic medical implants and in particular to a medical implant comprising a cup prosthesis, with an attachment member for attachment to an instrument head, and an instrument head. The present invention also relates to an assembly of the medical implant and an instrument head.

Orthopaedic hip surgery is known. During such surgery, an implant may be used to line to the acetabulum of a patient's pelvis and provide a concave surface for articulation of the hip joint. Such an implant has a general form in the shape of a cup or bowl and is often referred to as a cup prosthesis or as an acetabular cup prosthesis.

A number of different forms have been proposed for a medical implant that can function as an acetabular cup prosthesis. One example is discussed in EP-2174621 A1, which relates to a prosthesis comprising a ceramic acetabular cup and a metal band applied around the outer circumference of the cup adjacent to the rim of the cup. A ceramic acetabular cup has good wear characteristics and has good compressive strength. The metal band provides increased strength to the acetabular cup; in particular it increases the hoop compression on the cup prosthesis so that it is pre-stressed. This increases the strength of the prosthesis and reduces the likelihood of fracture when exposed to impaction forces during implantation.

WO-2010/146398 A1 discusses an acetabular cup prosthesis with a metal band applied around its circumference adjacent to the rim of the cup. In order to provide more secure attachment of the prosthesis to an impaction instrument, flanges extend from the metal band to connect to an impaction head.

In one embodiment of WO-2010/146398 A1, the flanges are elongated strips which are bent over the impaction head and secured close to the axial centre of the radial head. In another embodiment, one or more flanges extend upwardly from the metal band to engage an impaction head. In some of the embodiments, each flange includes an aperture in a free end for receiving a respective projection at the centre of an impaction cap extending in an axial direction. In all the embodiments, the flanges include a thinner region which is designed to be frangible and broken once the prosthesis has been implanted, thereby releasing the instrument head from the prosthesis.

The thinner region is broken by rotating the instrument cap relative to the prosthesis after implantation. The instrument cap includes notches or lugs which apply pressure to the flanges when rotated, causing them to break at their weakest point, the position of the thinner region.

The prosthesis of WO-2010/146398 A1 has a number of disadvantages. In the embodiment where the strips are elongated and bent over the impaction head, there is a risk that the flange will break prematurely at the thinner region during the bending. If the flange does break prematurely, a secure connection to an impaction head is not possible and the implant is wasted.

In the embodiment of WO-2010/146398 with upwardly extending flanges to engage the impaction head, it is not necessary to bend to flanges. However, it is difficult to engage such upwardly extending flanges reliably with the impaction head.

Accordingly, the present invention provides an assembly comprising: a medical implant comprising: a cup prosthesis having a circumference and a central axis which defines an axial direction and a radial direction; and an attachment member applied around the circumference of the cup, wherein the attachment member comprises: a circumferential band; and at least two tabs extending in the axial direction from the circumferential band and having a circumferential region of weakness, each of the at least two tabs delimiting an opening; and an instrument head attached to the attachment member and engaged with the cup prosthesis, the instrument head comprising an axial retaining element extending from the instrument head and engaged with the opening of the at least two tabs, characterised in that the axial retaining element extends from the instrument head in the radial direction and each of the at least two tabs delimit an opening in the radial direction adapted for engagement by the radially extending axial retaining element.

The radial opening allows the tab to be attached securely to the instrument head and, because it is a radial opening for engaging a radially extending element, no bending of the tab is required. Surprisingly, the presence of the radial opening in the tab does not weaken the tab unduly to axial forces, so that the radial tab retains its integrity during the implantation procedure (in which the medical practitioner may apply forces as high as 35 kN).

The instrument head is attached to the attachment member by the axial retaining element engaging the opening in the at least two tabs. This provides an effective attachment without the need to bend the tabs. The instrument head is also engaged with the cup prosthesis to allow forces to be transferred to the cup prosthesis by the instrument head.

The radial direction is analogous to the radial dimension *r* and the axial direction is analogous to the vertical direction z in cylindrical polar coordinates.

The at least two tabs may be integrally formed or non-integrally formed with the circumferential band. It is preferred that they are integrally formed and the circumferential region of weakness can then be provided by a thinned region of the tab adjacent the circumferential band to facilitate separation of the tabs from the band after implantation. Alternatively, if tabs are not integrally formed with the band, they may be bonded to the band so that bond forms the circumferential region of weakness.

The cup prosthesis is preferably manufactured from a ceramic material, or a material with ceramic properties. The circumferential band is preferably manufactured from a metal or metal alloy.

The opening in the radial direction allows the tabs to be engaged securely by an instrument head without requiring bending of the tabs.

In use, the implant is attached to an instrument head using the attachment member. After implantation, the tabs are separated from the circumferential band by breaking the tabs in the region of weakness. The circumferential band remains in place to provide additional strength to the implant.

Preferably, each of the at least two tabs delimits an opening which has a substantially flat portion parallel to the circumferential band and a tapering portion which tapers in the direction towards the circumferential band. The tapering portion preferably also tapers in the direction towards the apex of the cup prosthesis. This form of opening provides better distribution of the forces applied by an attached instrument head. A medical practitioner may not apply purely axial forces to the prosthesis as it is implanted. Typically the forces will include radial or circumferential components that may vary, creating a "wobbling" or "toggling" in the force applied. This shape of opening can distribute the load from such forces more evenly.

Each of the at least two tabs may delimit an opening which is generally triangular. This can help in ensuring that forces during impaction are evenly distributed. Preferably, each of the at least two tabs delimits an opening which is generally triangular and has two sides of equal length that define the tapering portion.

The tapering portion preferably tapers to a rounded apex. The rounded apex may have any form but is preferably symmetrical. In one embodiment the rounded apex defines a portion of a radius of a circle, and other embodiments may have other forms. A rounded apex helps to avoid stress concentrations while allowing the load distribution benefits of a tapered opening.

Preferably, the circumferential band has a height in the axial direction and each of the at least two tabs extend in the axial direction for a distance which is less than or equal to the height of the circumferential band. This construction means that the tab is relatively short in comparison to the band. Shorter tabs are simpler and easier to manufacture, required less material and less complicated manufacturing techniques than more elongated tabs.

The tabs can be located at a plurality of different positions around the circumference of the circumferential band. One or a plurality of tabs can be provided at each of the plurality of different positions. The plurality of different positions can be substantially equi-angularly spaced around the circumference.

The attachment member can comprise at least three tabs. Each of the tabs can have any of the preferred features discussed above. The three tabs can be located at three different positions around the circumference of the circumferential band. The three different positions can be substantially equi-angularly spaced. The three different positions can be at 120° intervals.

The attachment member can include one or a plurality of axial ribs. The or each axial rib can extend from the circumferential band. There can be at least three axial ribs. When there is a plurality of axial ribs, they can be spaced substantially equi-angulalry around the circumferential band. The or each axial rib can protrude from the circumferential band in the radial direction and/or extend in the axial direction.

The axial retaining element may be formed from a single piece with at least two radial extensions to engage respective openings in the at least two tabs. For example, the axial retaining element may comprise a circumferential member centred on the axis of the instrument head, with radial extensions to engage the openings.

In an alternative embodiment, the axial retaining element may comprise a plurality of discrete members, each of the discrete members engaging at least one of the openings.

The axial retaining element can be moveable in a radial direction, in particular moveable in a radial direction towards the axis. This allows the axial retaining element to be moved towards the axis and out of engagement with the opening in the at least one tab, allowing removal of the instrument head from the attachment member without requiring breaking of the region of reduced strength.

The axial retaining element may be biased in an outward radial direction. This can be provided by a resilient member, for example a spring or elastomer in one embodiment. In other embodiments, the biasing may be provided by the natural resilience of the material forming the axial retaining element.

The instrument head can form part of an impaction or insertion instrument, or be configured for connection to an impaction or insertion instrument.

Embodiments of the invention will now be described by way of example only and not limitation with reference to the accompanying drawings, in which:
Figure 1 depicts a perspective view of a surgical instrument head assembled with a cup prosthesis and attachment member according to a first embodiment of the invention;
Figure 2 depicts a top view of the assembly of Figure 1;
Figure 3 depicts a side view of the assembly of Figure 1;
Figure 4 depicts a cross-section along the line A-A in Figure 3;
Figure 5 depicts an exploded view of the assembly of Figure 1;
Figure 6 depicts a perspective view of a top portion of a surgical instrument head used in the assembly of Figure 1;
Figure 7 depicts a side view of the top portion of an instrument head depicted in Figure 6;
Figure 8 depicts a cross-section along line A-A in Figure 7;
Figure 9 depicts a perspective view of a bottom portion of a surgical instrument head used in the assembly of Figure 1;
Figure 10 depicts a side view of the bottom portion of the surgical instrument head depicted in Figure 9;
Figure 11 depicts a cross-section along line A-A in Figure 10;
Figure 12 depicts a perspective view of a tab separation member used in the assembly of Figure 1;
Figure 13 depicts a top view of the tab separation member depicted in Figure 12;
Figure 14 depicts a side view of the tab separation member depicted in Figure 12;
Figure 15 depicts a perspective view of an axial retaining element used in the assembly of Figure 1;
Figure 16 depicts a top view of the axial retaining element of Figure 15;
Figure 17 depicts a perspective view of an attachment member used in the assembly of Figure 1;
Figure 18 depicts a side view of the attachment member of Figure 17;
Figure 19 depicts a cross-section through line A-A in Figure 18;
Figure 20 depicts a perspective view of an assembly of a surgical instrument head, cup prosthesis and attachment member according to a second embodiment of the invention;
Figure 21 depicts a top view of the assembly of Figure 20;
Figure 22 depicts a side view of the assembly of Figure 20;
Figure 23 depicts a cross-section along line A-A in Figure 22;
Figure 24 depicts an exploded view of the assembly of Figure 20;
Figure 25 depicts a perspective view of a tab separation member used in the assembly of Figure 20;
Figure 26 depicts a top view of the tab separation member of Figure 25;

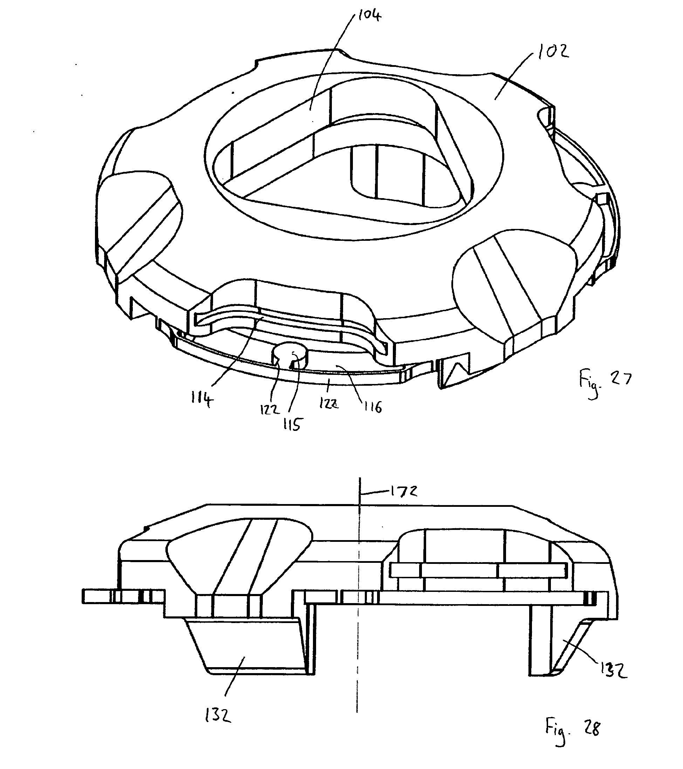

Figure 27 depicts a perspective view of a surgical instrument head used in the assembly of Figure 20;
Figure 28 depicts a side view of the surgical instrument head of Figure 27;
Figure 29 depicts a perspective view of an axial retaining element used in the assembly of Figure 20;
Figure 30 depicts a top view of the axial retaining element of Figure 29;
Figure 31 depicts a perspective view of a resilient element used in the assembly of Figure 20;
Figure 32 depicts a top view of the resilient element of Figure 31;
Figure 33 depicts a side view of the resilient element of Figure 31;
Figure 34 depicts a perspective view of an assembly of a surgical instrument head, cup prosthesis and attachment member according to a third embodiment of the invention;
Figure 35 depicts a top view of the assembly of Figure 34;
Figure 36 depicts a side view of the assembly of Figure 34;
Figure 37 depicts a cross-section along line A-A in Figure 36;

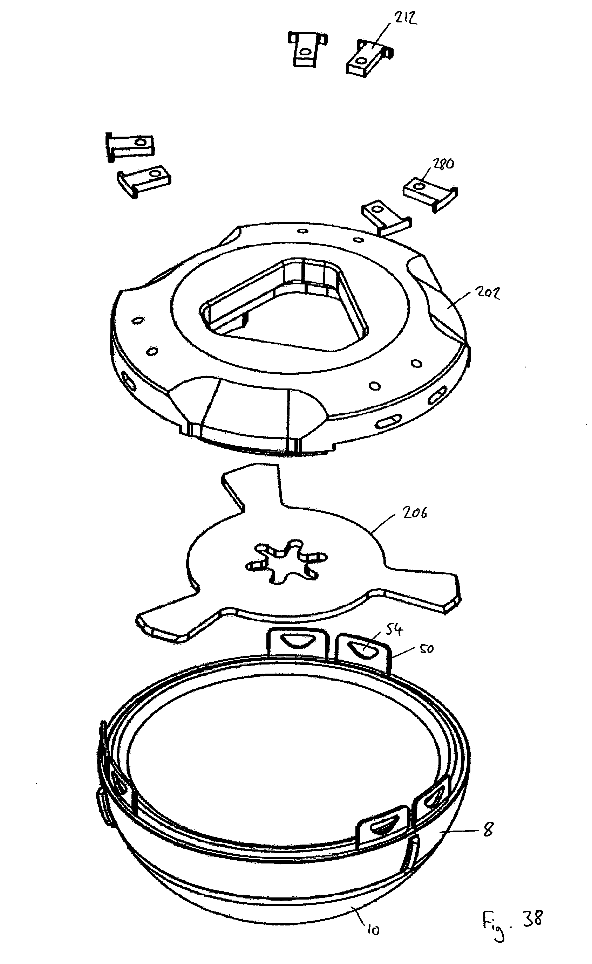

Figure 38 depicts an exploded view of the assembly of Figure 34;
Figure 39 depicts a perspective view of a surgical instrument head used in the embodiment of Figure 34;
Figure 40 depicts a side view of the surgical instrument head of Figure 39;
Figure 41 depicts a perspective view of a tab separation member used in the assembly of Figure 34;
Figure 42 depicts a top view of the tab separation member of Figure 40;
Figure 43 depicts a perspective view of a surgical instrument for rotating the tab separation member relative to the surgical instrument head;
Figure 44 depicts a top view of the surgical instrument of Figure 43;
Figure 45 depicts a cross-section along line A-A in Figure 43; and
Figure 46 depicts a top view of the surgical instrument of Figure 43.

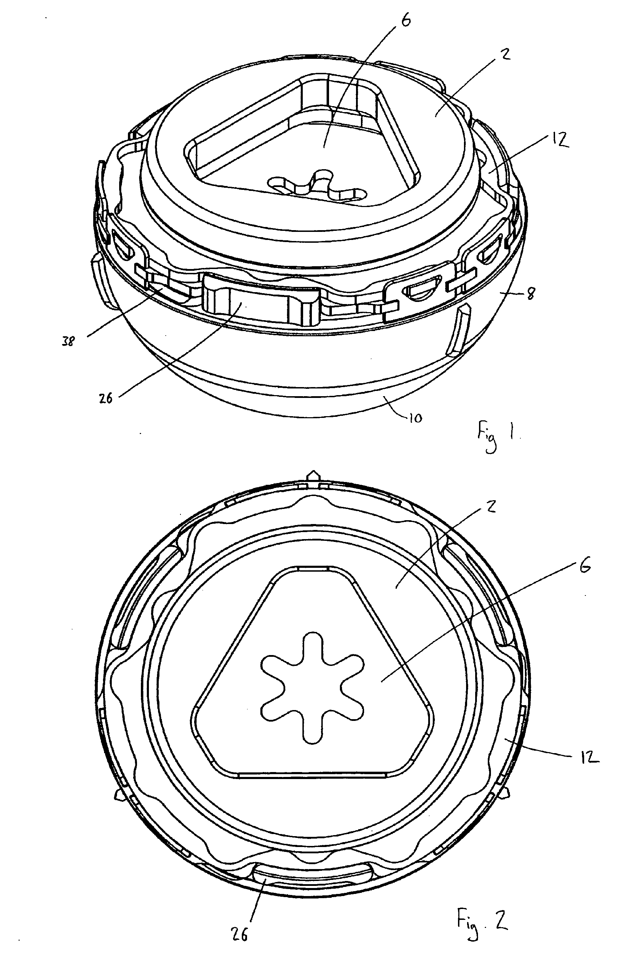

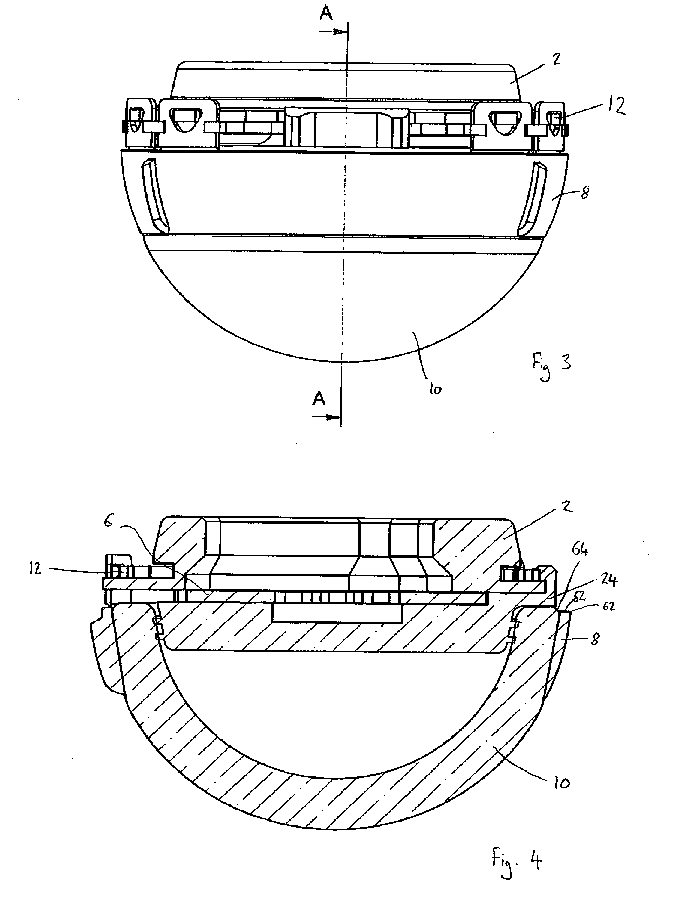

Figure 1 depicts a perspective view of an assembly of a surgical instrument head, cup prosthesis and attachment member according to a first embodiment of the present invention. Figure 2 depicts a top view of the assembly of Figure 1. Figure 3 depicts a side view of the assembly of Figure 1 and Figure 4 depicts a cross-section along line A-A in Figure 3. The assembly comprises a surgical instrument head, which comprises an upper part 2 and a lower part 24. A tab separation member 6 is contained within the surgical instrument head. An attachment member 8 is applied to a cup prosthesis 10. The surgical instrument head is engaged with the attachment member 8, and axially retained relative to the attachment member 8 using an axial retaining element 12.

As can best be appreciated with reference to the side view of Figure 3, the components of the assembly of Figure 1 are assembled coaxially, about a common axis defined by the cup prosthesis 10. This axis extends from the apex of the cup prosthesis 10 and defines an axial direction along the axis and a radial direction extending outwardly from the axis. The line A-A in Figure 3 follows the line of this central axis.

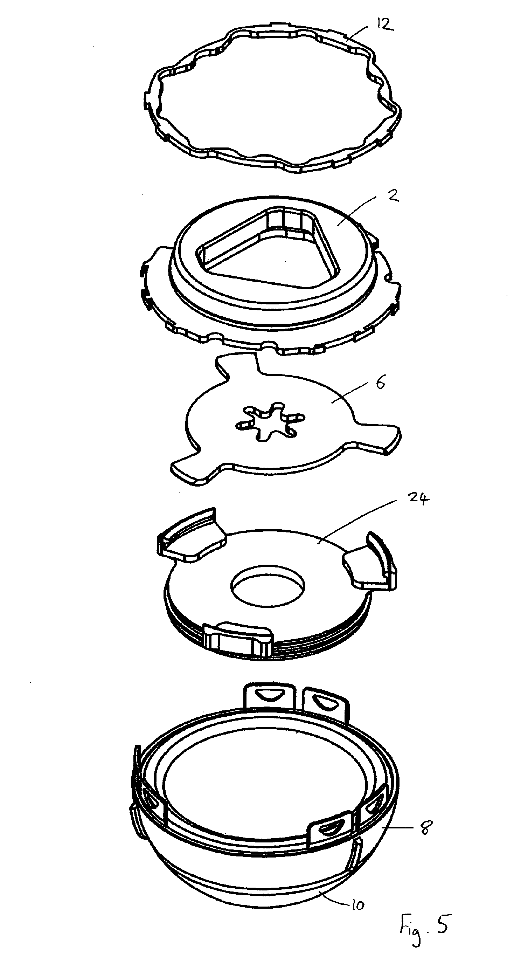

Figure 5 depicts an exploded view of the assembly of Figure 1. The construction of the individual components within the assembly of Figure 1 will now be described in more detail.

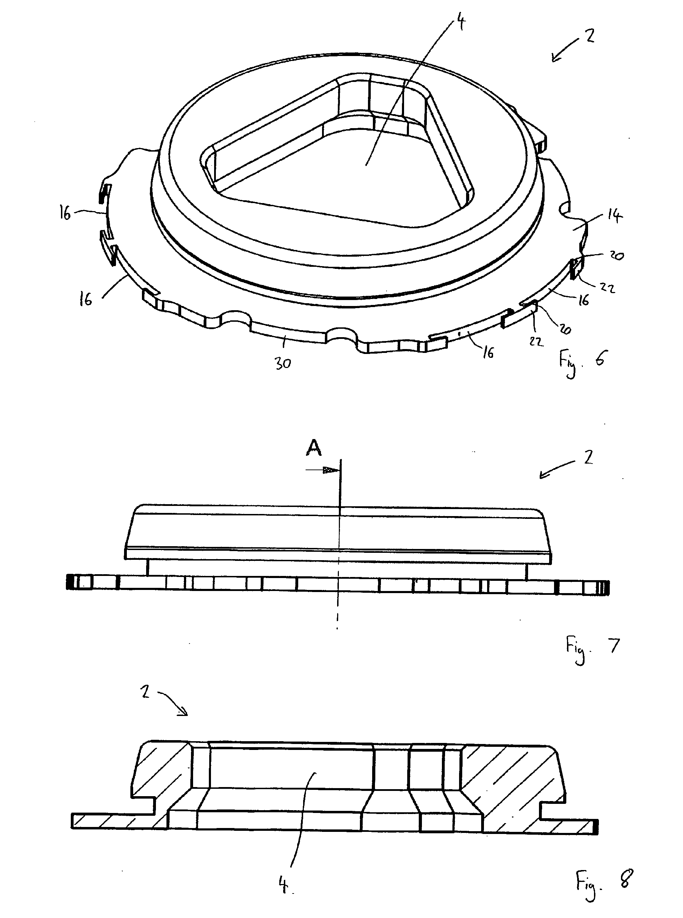

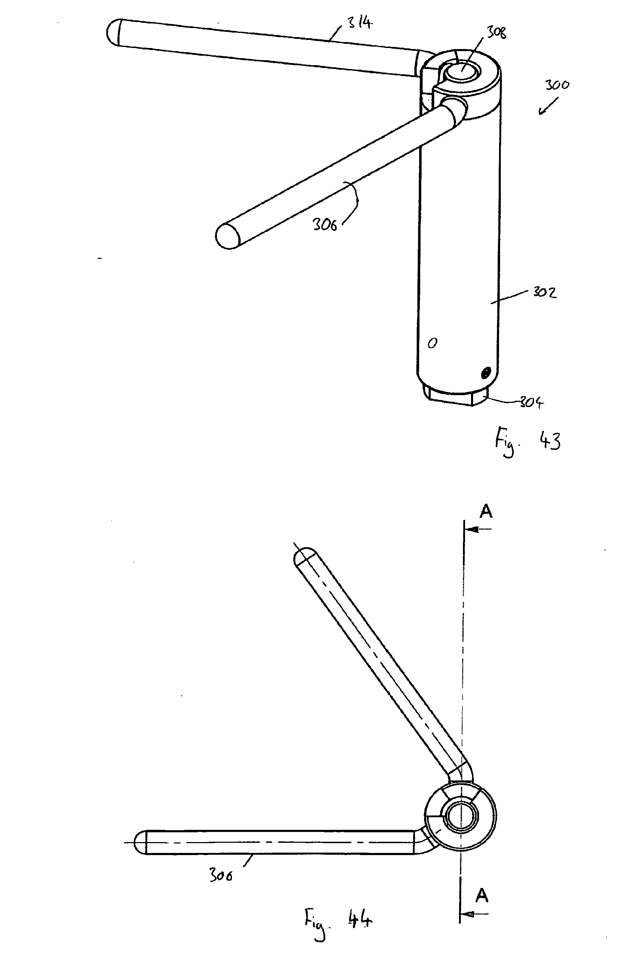

Figure 6 depicts a perspective view of a top part 2 of a surgical instrument head. The top part is preferably machined from a polymer, metal or metal alloy, for example 316 stainless steel. The top part 2 of the surgical instrument head defines an opening 4 which provides a generally triangular formation for engagement by a surgical instrument, allowing relative rotation of the surgical instrument head and the tab separation member 6. This surgical instrument will be described in more detail later, with reference to Figures 43 - 46. As can be seen in Figure 6 and in Figure 2, the formation is in the form of a truncated triangle which, when assembled is centred on the axis of the cup prosthesis 10.

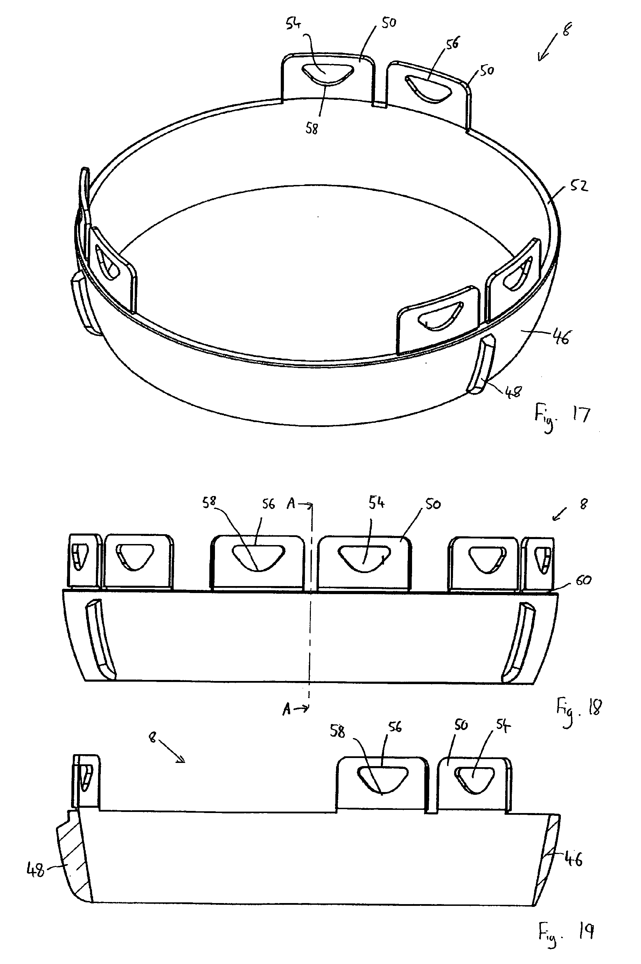

The top part 2 of the surgical instrument head also comprises a generally planar flange 14 which is parallel to the radial direction, perpendicular to the central axis. The flange 14 defines axially oriented slots 16 for receiving tabs of the attachment member 8 (the tabs of the attachment member 8 are described in more detail later with reference to Figures 17-19). The axially extending slots 16 include a face 20 which follows a line extending radially from the central axis. The face 20 can engage the side of a tab of the attachment member 8 to prevent relative rotation of the attachment member to the instrument head. The axially extending slots 16 also comprise a protrusion 22 which engages an outer circumferential face of the tab of the attachment member 8. These protrusions 22 function as a radial retaining element to prevent a tab from moving in the outward radial direction when the surgical instrument head is assembled and attached to an attachment member 8 of a cup prosthesis 10.

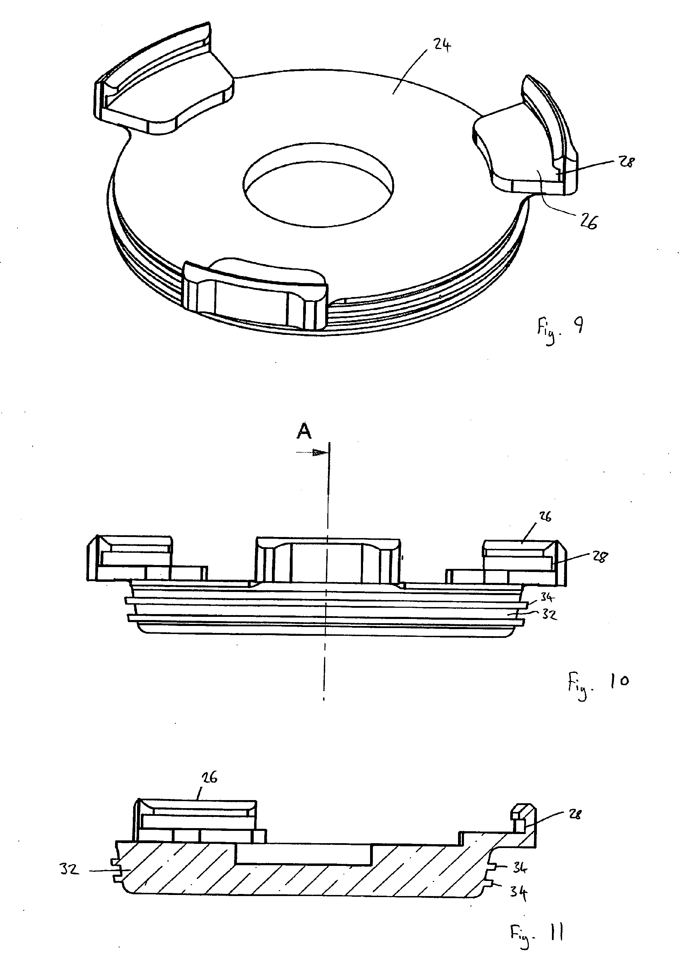

Other portions of the circumference of the flange 14 are indented in a radial direction relative to the axial slot 16. These engage discrete upwardly extending flanges 26 formed on a lower part 24 of the surgical instrument head which is depicted in perspective view in Figure 9. A side view of the lower part 24 is depicted in Figure 10, and a cross-section is depicted in Figure 11. As can be seen in Figure 9, the lower part 24 of the surgical instrument head comprises three flanges 26 which are radially spaced 120° apart about the central axis. These flanges 26 extend from an upper portion of the lower part 24. The flanges 26 define a partially circumferential slot 28 which follows a circumference centred on the central axis. This slot 28 receives the recessed portion 30 of the top part 2 of the surgical instrument head, to connect the top part 2 to the lower part 24 and prevent relative movement along the central axis and rotation about the central axis between the top part 2 and the lower part 24.

The lower part 24 also comprises a tapered member 32 extending below the flanges 26. This tapered member 32 extends continuously around a circumference centred on the central axis and tapers to a narrower diameter in the downward direction (towards the cup prosthesis when assembled). The tapered member 32 is provided with two circumferential ribs 34.

The diameter of the tapered member 32 is manufactured to be slightly less than the internal diameter of the cup prosthesis 10 (seen most clearly in Figure 4). However, the circumferential ribs 34 have a diameter which is larger than the internal diameter of the cup prosthesis 10. The ribs 34 are configured to deform plastically when the lower part 24 of the surgical instrument head is inserted into a cup prosthesis 10. This plastic deformation provides a tight fit, without requiring undue force. The continuous circumferential nature of the ribs provides a good seal around the entire circumference of the cup prosthesis 10.

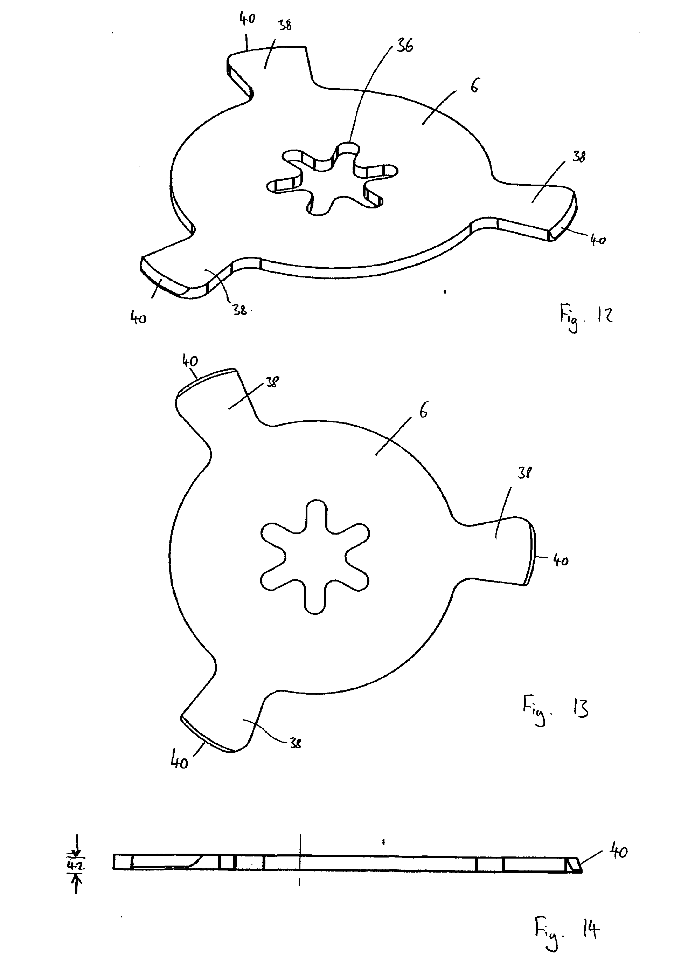

Referring now to Figure 12, a perspective view of a tab separation member 6 is depicted. A top view is shown in Figure 13 and a side view in Figure 14.

The tab separation member 6 comprises a formation 36 configured to engage a portion of a surgical instrument for relative rotation of the tab separation member 6 to the surgical instrument head. The formation 36 is generally star shaped and similar to the shape of the typographical character for an asterisk in this embodiment, although other shapes may also be used. The rotation instrument will be described later in more detail with reference to Figures 43 - 46.

The tab separation member 6 is provided with three protrusions 38 extending in a radial direction which each provide a cam surface 40 at their outer radial edge. The cam surface 40 is no concentric with the central axis of the tab separation member. The dimensions of the cam surface 40 are chosen such that the cam surface 40 is at a radial position slightly further from the central axis that the tabs of the attachment member 8 defined in more detail later.

The protrusions 38 are spaced 120° apart about the central axis and the tab separation member 6 has rotational symmetry about the central axis. As can be seen most clearly in Figures 12 and 14, the tab separation member 6 has a thickness 42 in the axial direction. This thickness 42 is required to give the tab separation member 6 sufficient mechanical strength. In order to concentrate the force applied by the cam edge 40 on a tab of the attachment member 8, the cam edge 40 is tapered in an upward direction, so that the radial dimension of the protrusion 38 is greater at a lower portion of the tab separation member 6 than an upper portion (the radial dimension is greater in the direction towards the cup prosthesis when assembled). Preferably, this edge is slightly rounded rather than sharp and therefore can be considered similar to a blunt knife edge.

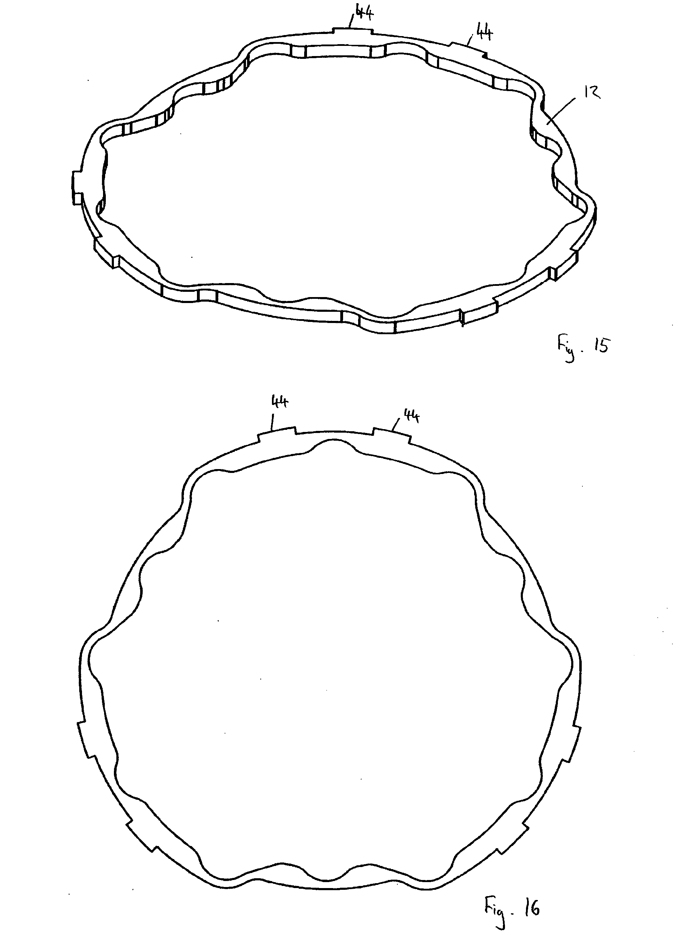

Referring to Figure 15, a perspective view of the axial retaining element 12 is depicted. Figure 16 depicts a top view of this axial retaining element 12. The axial retaining element 12 is received on top of the flange 14 of the top part 2 of the surgical instrument head. It is manufactured from a resilient material, for example a metal or metal alloy, preferably stainless steel, for example that sold under the trade name 17/4 PH and processed to Condition H900 commercially available from AK Steel, Ohio, USA.. As can be seen most clearly in Figure 16, the axial retaining element 12 has rotational symmetry about the central axis. It comprises protrusions 44 extending in a radial direction for engaging openings in the tab of the attachment member 8 (the attachment member 8 is described in more detail below).

The axial retaining element 12 has the form of a continuous, generally circumferential member or ring which has a relatively thin radial thickness compared to its radius. This enables the axial retaining element 12 to be deformed radially inward if required. In the absence of an external force, the natural resilience of the material causes it to take the form depicted in Figures 15 and 16, with the radial protrusions 44 located about the central axis with rotational symmetry. In this embodiment, there are six radial protrusions 44 to engage the openings in six tabs of the attachment member 8. However, in other embodiments a different number of protrusions 44 may be provided, depending on the number of tabs in the attachment member 8.

Figure 17 depicts a perspective view of an attachment member 8. A side view is depicted in Figure 18 and a cross-section depicted in Figure 19.

The attachment member 8 comprises a circumferential band 46 which is applied around the circumference of the cup prosthesis 10. The circumferential band 46 comprises axial ribs 48 extending from it. Three axial ribs 48 are provided, spaced 120° apart about the central axis with rotational symmetry. In other embodiments, different numbers and arrangements of axial ribs 48 may be used.

In use, the circumferential band 46 remains in place attached to a cup prosthesis 10 once the cup prosthesis 10 has been implanted into a patient. The axial ribs 48 assist the implantation to the patient. In the cross-section of Figure 19, the left-hand side intersects one of the axial ribs 48.

The attachment member includes a plurality of tabs 50 which extend upwardly from an upper face 52 of the circumferential band 46. In this embodiment six tabs are provided spaced in groups of 2 at 120° intervals around the central axis. In other embodiments, different arrangement of tabs may be used.

The tabs 50 are adapted to be engaged by portions of the surgical instrument head and the axial retaining element 12 to attach the cup prosthesis 10 (to which the circumferential band 46 is attached) to the instrument head.

To facilitate this attachment, the tabs 50 each define an opening 54 which has a generally triangular shape. An upper surface 56 of the opening 54 is generally planar and parallel to a plane perpendicular to the central axis. This face 56 can therefore be engaged by a corresponding surface of the protrusion 44 of the axial retaining member 12 to prevent relative axial movement between the attachment member and the axial retaining member and therefore also prevent relative axial movement between the cup prosthesis 10 and the instrument head.

The opening 54 tapers downwardly (in the direction towards the cup prosthesis when assembled) with a generally triangular shape, leading to a rounded apex 58. This shape of opening 54 helps to distribute the forces exerted on the attachment member 8 during impaction, and initial surgical assessment of fixation of the cup prosthesis 10 into a patient. The symmetrical tapering and the rounded apex 58 distribute the forces evenly around the tab 50, avoiding stress concentrations and helping to avoid premature breakage of the tabs 50 from the circumferential band 46.

The tabs 50 are provided with a weakened circumferential region 60. In this embodiment, the tabs 50 are integrally formed with the circumferential band 48. A region of weakness 60 is provided by thinning the material of the tab in the portion adjacent to the upper face 52 of the circumferential band 46. This thinned area can be relatively easily broken to separate the tab 50 from the circumferential band 48 by the application of an outward radial force.

As can be seen most clearly in Figure 4, the upper face 52 of the circumferential band 46 has an outer circumferential edge 62, which is rounded to reduce potential trauma to the patient. The circumferential band 46 is mounted to the cup prosthesis 10 so that its upper face 52 is at an axial position which is lower than the outer edge 64 of the rim of the cup prosthesis 10. The outer edge 64 of the cup prosthesis 10 is also rounded. As can be seen in Figure 4, most clearly at the left-hand side, the rounded outer edge 62 of the upper face 52 and the rounded rim 64 of the cup prosthesis define a slight recess between them. The region of weakness 60 is positioned adjacent to the upper face 52 of the circumferential band 46 and therefore is located within this recess. Therefore, when the tab 50 is broken from the circumferential band 46, any rough edges remaining are located within the recess and shielded from a patient.

To assemble the embodiment of the present invention, the tab separation member 6 is sandwiched between the upper part 2 and the lower part 24 of the instrument head.

The attachment member 8 is attached to a cup prosthesis 10 by a thermal differential assembly process. It is preferred that the cup prosthesis 10 is manufactured from a ceramic or ceramic-like material and that the attachment member 8 is manufactured from metal or a metal alloy, for example titanium or a titanium alloy. In that case the attachment member can be press-fitted by heating the attachment member 8 using induction heating. The induction heating will not affect the ceramic cup prosthesis 10 and can be carried out by any suitable means. Typically, the attachment member 8 will be heated to about 350°C. Once fitted, the induction heating is removed and the attachment member will cool, shrinking and increasing the pre-stress applied to the cup prosthesis.

The attachment member 8 is slid into the assembled instrument head so that the tabs 50 align with the axial slots 16. The instrument head is then pressed into the cup prosthesis 10, so that the tapering member 32 functions as a locating member and is received against an inside face of the cup prosthesis 10. The deformable ribs 34 deform plastically against the surface of the cup prosthesis. This provides a tight fit and reduces the effect of tolerance stack up of the manufactured components. The ribs 34 align the central axis of the instrument head with the central axis of the cup prosthesis.

Finally, the protrusions 44 of the axial retaining element 12 are inserted into the holes 54 of the tabs 50. Thus, the instrument head is securely attached to the cup prosthesis 10 with the attachment member 8. The tabs 50 are held securely so that they cannot move in axial or radial directions relative to the instrument head nor rotate about the central axis. The axial retaining element 12 prevents movement in an axial direction, the protrusions 22 of the axial slots 16 prevent movement in a radial direction and the faces 20, in addition to the fit of the protrusion 44 into hole 54, act to prevent rotation of the tab about the axis.

In use, it is desired to detach the tabs 50 from the circumferential band 46 along the region of weakness 60 after implantation. The circumferential band 46 then remains in place on the implanted cup prosthesis to provide additional strength while the tabs 50 are removed with the instrument head.

Tabs 50 are broken by rotating the tab separation member 6 relative to the instrument head. This can be achieved using a surgical instrument as described later with reference to Figures 43 - 46. Rotation of the tab separation member engages the region of weakness with the cam edge 40. As the tab is restrained above the region of weakness by the protrusions 22, the tab cannot move outwardly in a radial direction in response to this force. Instead, the tab shears away from the circumferential band 46 in an outward direction. This detaches the tabs 50 from the circumferential band 46.

It is possible that sometimes a tab 50 may not completely detach from the circumferential band 46. In this situation, it is desirable to have a mechanism allowing manual detachment of the instrument head. In this case, manual detachment can be achieved by flexing the axial retaining member 12 in a radially inward direction, so that protrusions 44 disengage from holes 54. The instrument head can then be removed from any tabs remaining attached to the circumferential band by moving it in an axial direction. Any tabs remaining attached can then be broken away from the circumferential band by any other suitable means.

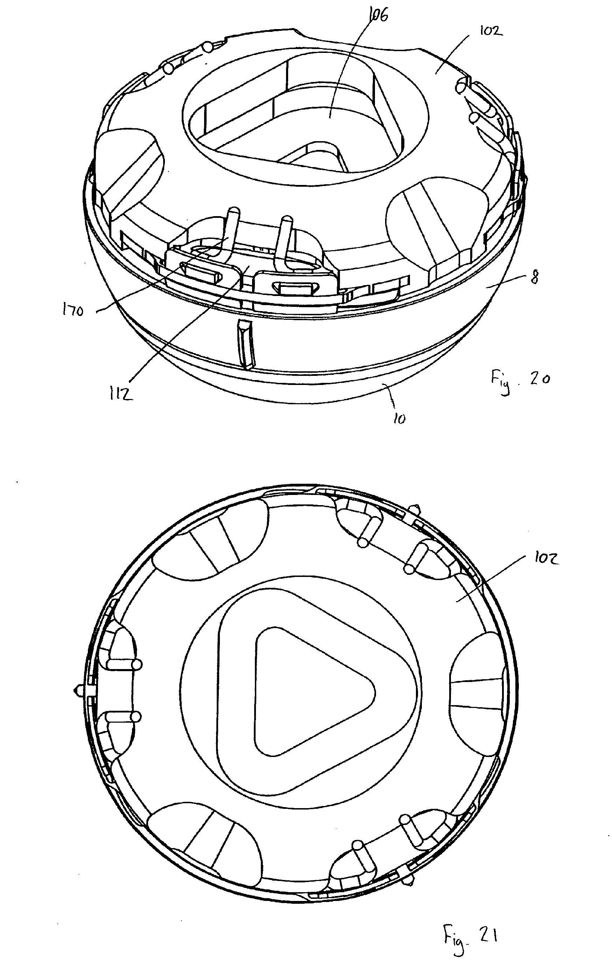

A second embodiment of an assembly of a surgical instrument head, cup prosthesis and attachment member is depicted in Figure 20. The construction of this embodiment is the same as the above-described first embodiment except as described below.

Figure 20 depicts a perspective view of an assembly comprising an instrument head 102, tab separation member 106, axial retaining element 112, resilient element 170, cup prosthesis 10 and attachment member 8.

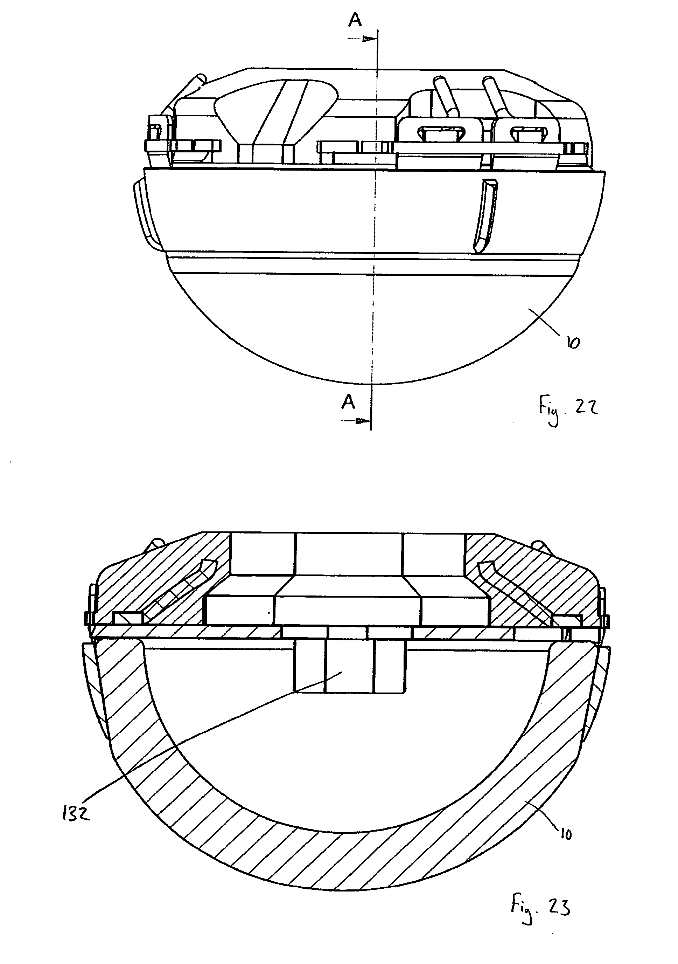

A top view of the assembly is show in Figure 21, Figure 22 depicts a side view and Figure 23 a cross-section along line A-A in Figure 22.

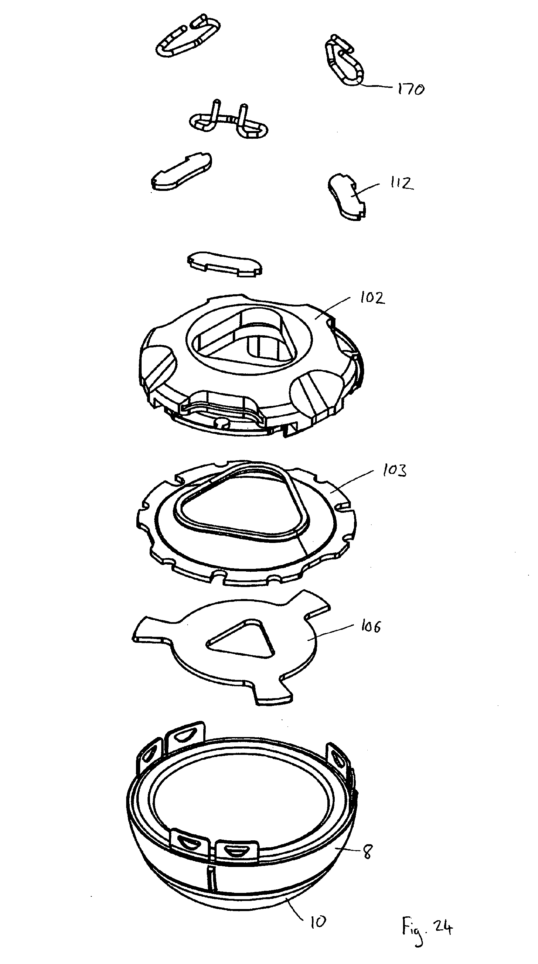

An exploded view of the assembly is depicted in Figure 24.

In this embodiment, the instrument head 102 is formed by injection moulding as a single piece. An upper portion of the surgical instrument head 102 defines an opening providing a formation 104 for engaging a rotation tool. The formation 104 is similar to the formation 4 described above with reference to Figure 6. However, in this embodiment, the formation 104 has a generally triangular shape with rounded corners of the triangle, so that it is more in the form of a rounded triangle than a truncated triangle.

A stainless steel plate 103 (visible in exploded view in Figure 24) is encapsulated within the injection moulded outer body that makes up the instrument head 102. The injection moulding takes place around the stainless steel plate 103. The stainless steel plate adds strength to the injection moulded plastic, allowing the component to withstand greater forces during implantation, and assessment of initial fixation.

The instrument head 102 defines axial openings 116 for receiving tabs from the attachment member 108. The slots 116 include an outer circumferential member 122 which engages an outer edge of the tab when assembled (seen most clearly in Figure 22). The axial slot 116 also include faces 120 which are parallel to the radial direction. In use, when a tab is received within slot 116, the member 122 prevents movement of the tab outwards in the radial direction and face 120 prevents relative rotation of the instrument head 102 relative to the tab about the central axis.

Instrument head 102 also defines a horizontal slot 114 which is parallel to a plane perpendicular to the central axis for receiving a retaining element 112 and a biasing element 170.

In order to ensure that the instrument head 102 is accurately aligned relative to the central axis 172, a plurality of locating members 132 extend downwardly from the instrument head 102 (in the direction towards the cup prosthesis when assembled). The locating members 132 are tapered in the direction away from the instrument head along the central axis, so that the external radial dimension of the locating element 132 is smaller in the direction towards the cup prosthesis. The locating elements 132 are arranged with rotational symmetry about the central axis 172. In this embodiment, three locating members 132 are provided which are spaced at 120° apart about the central axis 172. The taper angle of each of the locating members 132 is the same. The combination of these features enables the locating members 132 to accurately centre and align the central axis 172 of the instrument head 102 with the central axis of the cup prosthesis 110.

The instrument head 102 is preferably manufactured from a thermo setting polymer, for example nylon PA6. The locating members 132 are configured to deform plastically when the instrument head 102 is inserted into a cup prosthesis. This plastic deformation together with the rotational symmetry ensures that the instrument head achieves a tight, self-centred fit with the cup prosthesis so that its central axis is correctly aligned with the axis of the cup prosthesis 10.

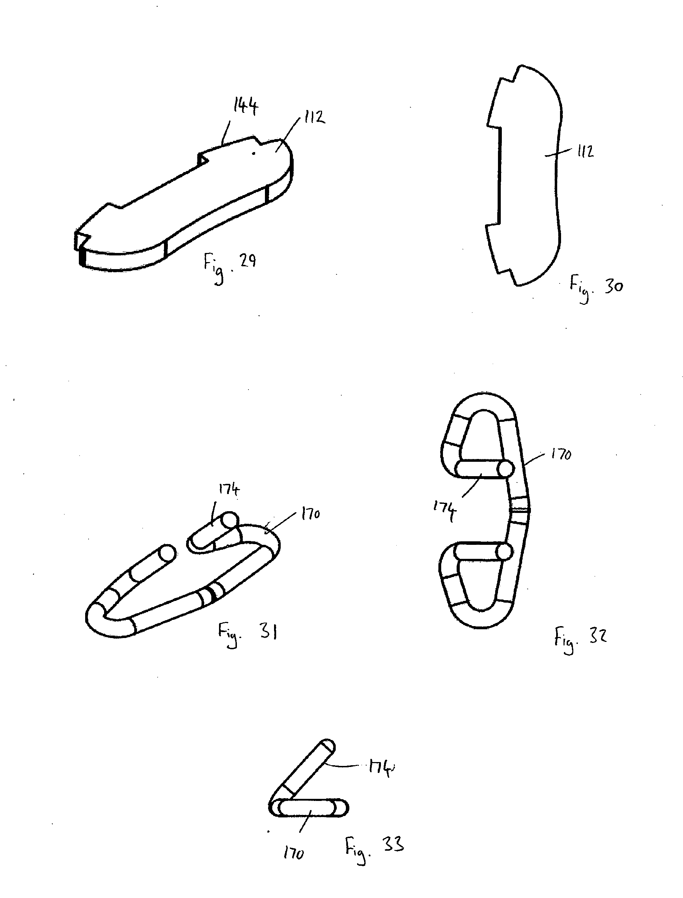

Referring to Figure 29, an axial retaining element 112 is depicted in perspective view. The top view is depicted in Figure 30. Unlike the single axial retaining element 12 described with reference to Figure 15, a plurality of discrete retaining elements are provided. As depicted in Figure 29, each retaining element includes two protrusions 144 for engaging holes formed in tabs of the attachment member. Three axial retaining elements 112 are provided, each received in a slot 114 of the instrument head and also supported by formation 115 extending inwardly from the radial retaining element 122. The axial retaining element 112 is biased in a radially outward direction by resilient element 170, depicted in perspective view in Figure 31, top view in Figure 32 and side view in Figure 33. Resilient element 170 is partially contained within slot 114 and includes free ends 174 which extend in an upward direction and resilient element 170 also engages the axial retaining element 112 to urge it outwardly.

The construction of the cup prosthesis 10 and attachment member 8 is the same as described above with reference to Figures 1-4 and 17-19.

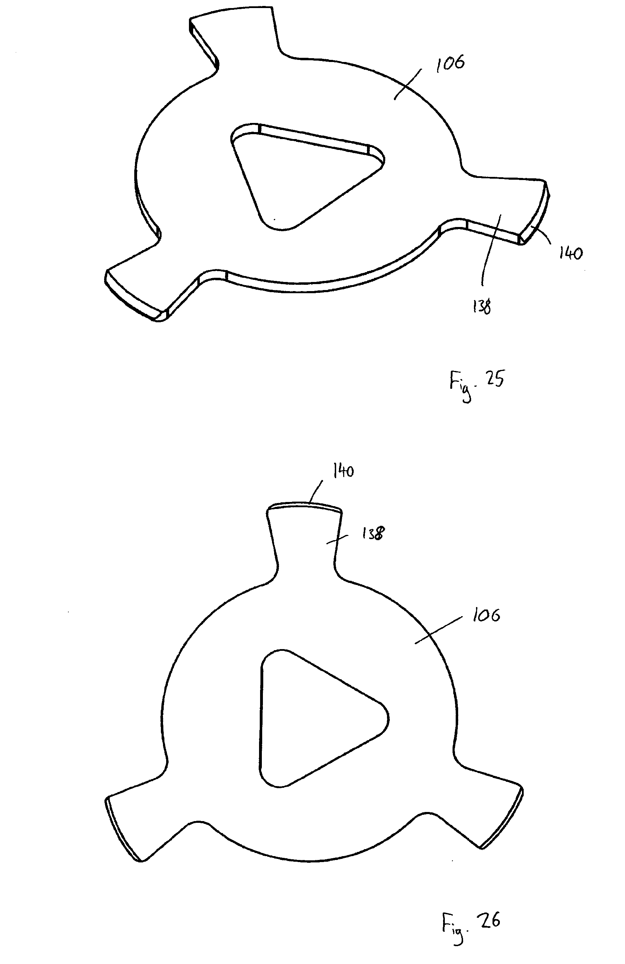

A tab separation member 106 is depicted in perspective view in Figure 25 and top view in Figure 26. This has generally the same construction as the tab separation member 6 described with reference to Figure 12, with three protrusions 138 each having a cam edge 140. The formation 136 for engaging a rotational tool is generally triangular in this embodiment.

The use and operation of this embodiment is generally as described above, with reference to Figures 1-19. The instrument head 102 is attached to the attachment member 8 by engaging the tabs 50 of the attachment member 8 with the axial slot 116 of the instrument head and engaging axial retaining element 112 with openings 54 of the tabs 50. When it is desired to disconnect the instrument head 102 from the attachment member 8, cam member 106 is rotated using a tool such as that described in Figures 43-46, to apply an outward force in the region of weakness of the tabs and break the tabs from the circumferential band.

Should the operation of the tab separation member 106 not work correctly, so that one or more of the tabs remain connected to the attachment member, this construction allows for manual disconnection of the instrument head by squeezing upwardly extending elements 174 of the resilient member 170 together. This deforms the member 170 in such a way that the outward force on the retaining element is removed, allowing the axial retaining element 112 to be slid radially inwards, disengaging protrusions 144 from openings 50.

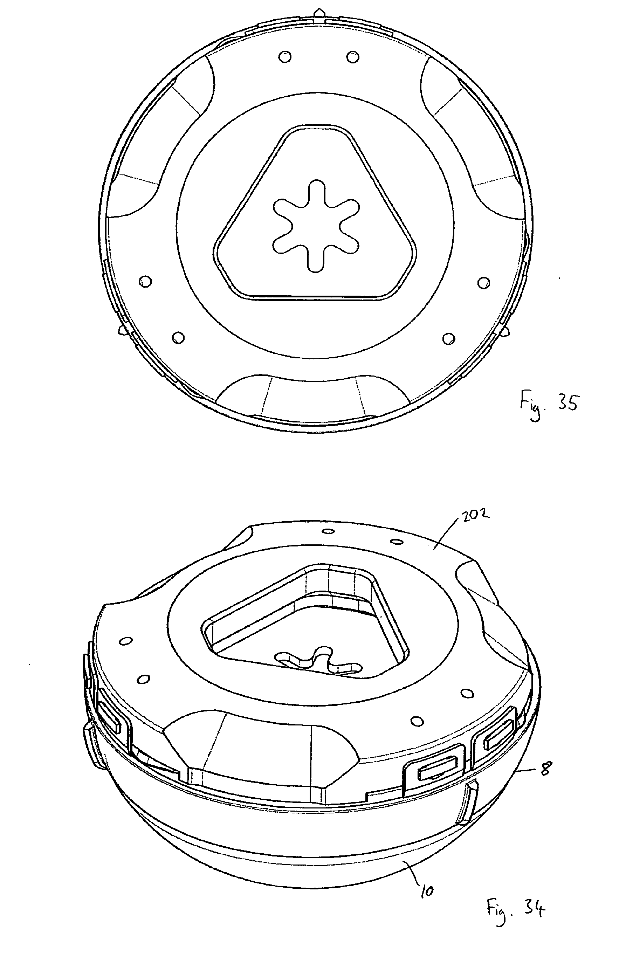

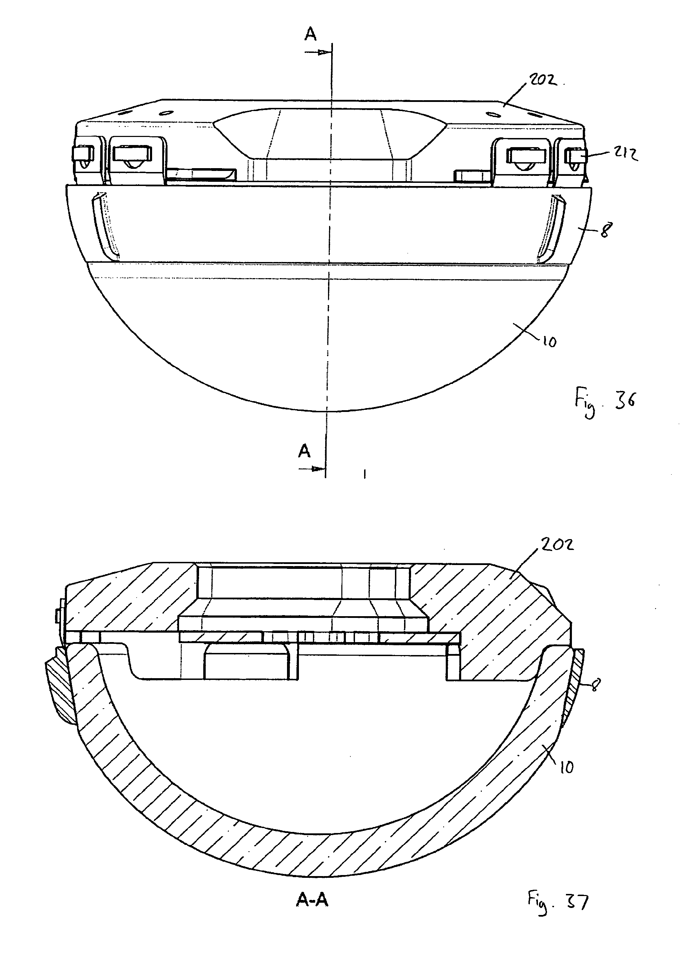

Figure 34 depicts a third embodiment of the present invention. A top view is depicted in Figure 35, a side view in Figure 36 and a cross-section in Figure 37. The construction of this embodiment is the same as the above-described first and second embodiments except as described below.

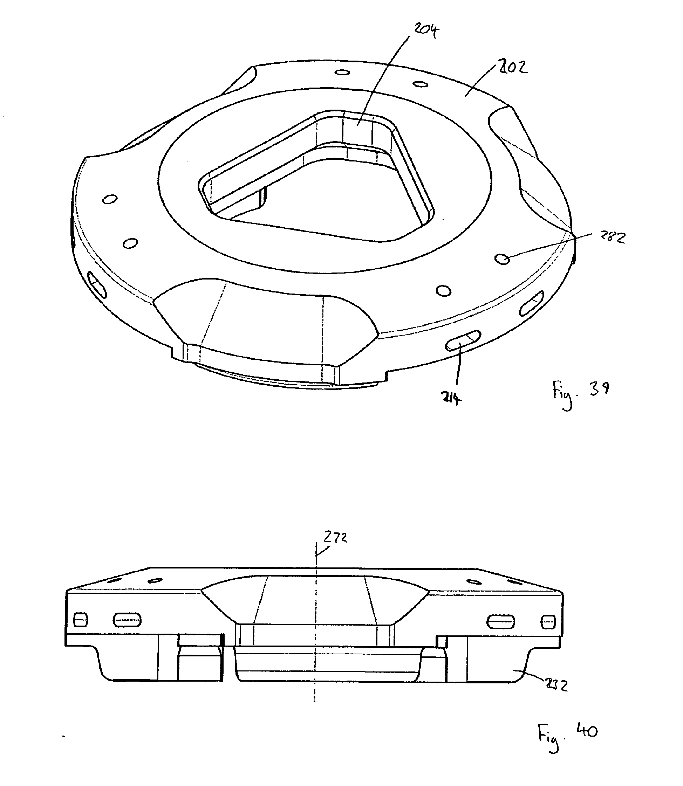

An instrument head 202 is formed as a single piece and is depicted in perspective view in Figure 39 and side view in Figure 40. Instrument head 202 includes horizontal slots 214 for receiving an axial retaining element 212. Instrument head 202 also defines an opening 204 having a formation for engaging a corresponding formation on a rotation instrument. The formation of the opening 204 is a truncated triangle, as described above with reference to Figure 6.

A stainless steel plate is preferably encapsulated within an injection moulded outer body that makes up the instrument head 202, in a similar way to the embodiment of Figure 24. The stainless steel plate adds strength to the injection moulded plastic, allowing the component to withstand greater forces during implantation, and assessment of initial fixation.

The surgical instrument head includes three tapered locating members 232 which extend axially downward from the surgical instrument head for engaging a cup prosthesis. The locating members 232 are preferably formed from nylon so that they deform plastically when the instrument head 202 is inserted into a cup prosthesis 10. The locating members 232 are provided with rotational symmetry about axis 272 so that a self-centering action is obtained to align axis 272 of the instrument head 202 with the axis of the cup prosthesis 10.

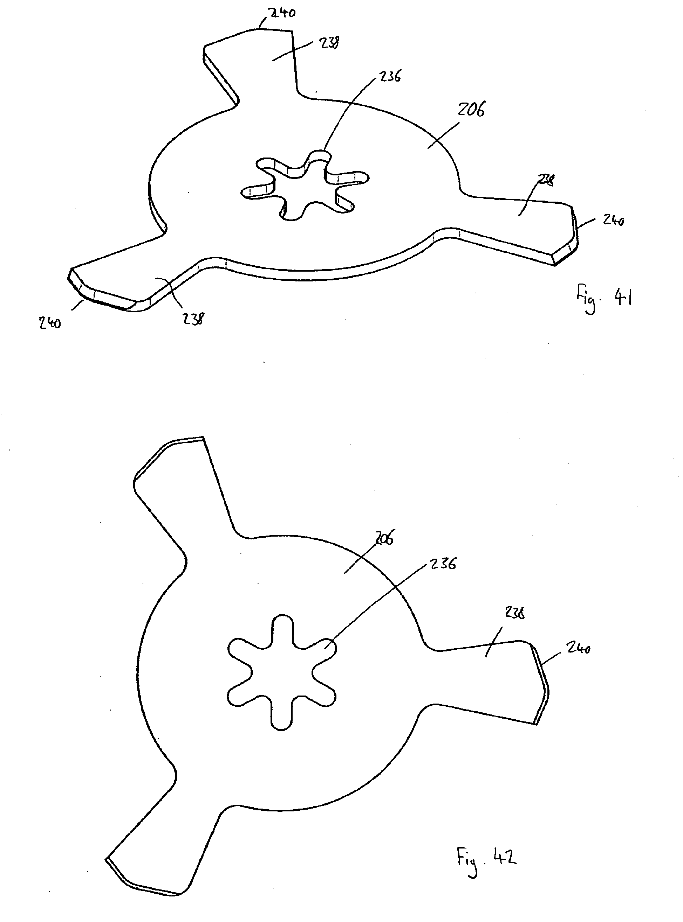

A tab separation member 206 is provided beneath the instrument head 202. The tab separation member 206 comprises three protrusions 238 defining cam surfaces 240 for engaging the weakened region of the tabs. A central opening 236 defines a generally star shaped formation for engaging a rotation instrument, such as the one described in more detail below with reference to Figures 43-46. The construction of tab separation member 206 is generally the same as the construction of tab separation member 6 described with reference to Figure 12. However, as can be seen by a comparison of Figures 41 and 42 with Figures 12 and 13, the profile of the cam edge 240 comprises two generally straight portions connected by a curve, rather than a single generally curved portion depicted with reference to Figures 12 and 13.

In use, the instrument head is assembled onto cup prosthesis 10 with attachment member 8 by placing it on the cup prosthesis so that slots 214 align with holes 54 in tabs 50. The retaining element 212 is then passed through hole 54 in tab 50 into slot 214 of the instrument head. The retaining element 212 includes an axial bore 280 which aligns with corresponding axial bores formed in the instrument head 202. The axial retaining element 212 can therefore be secured in the instrument head 202 by passing a rod or other cylindrical member through the bore 282 of the instrument head into the bore 280 of the radial retaining element 212.

In use, the tabs can be separated from the circumferential band by rotation of the tab separation member 206 relative to the instrument head 202. Should any of the tabs not be broken by this procedure, the tabs can be removed manually by removing the cylindrical member which retains the axial retaining elements 212 in the instrument head 202.

In this embodiment, the axial retaining element 212 also functions to prevent rotation of the instrument head relative to the attachment member, and as a radial retaining element preventing radial movement of the attachment member. Therefore, when the tabs are broken, the broken tabs remain securely attached to the instrument head 202.

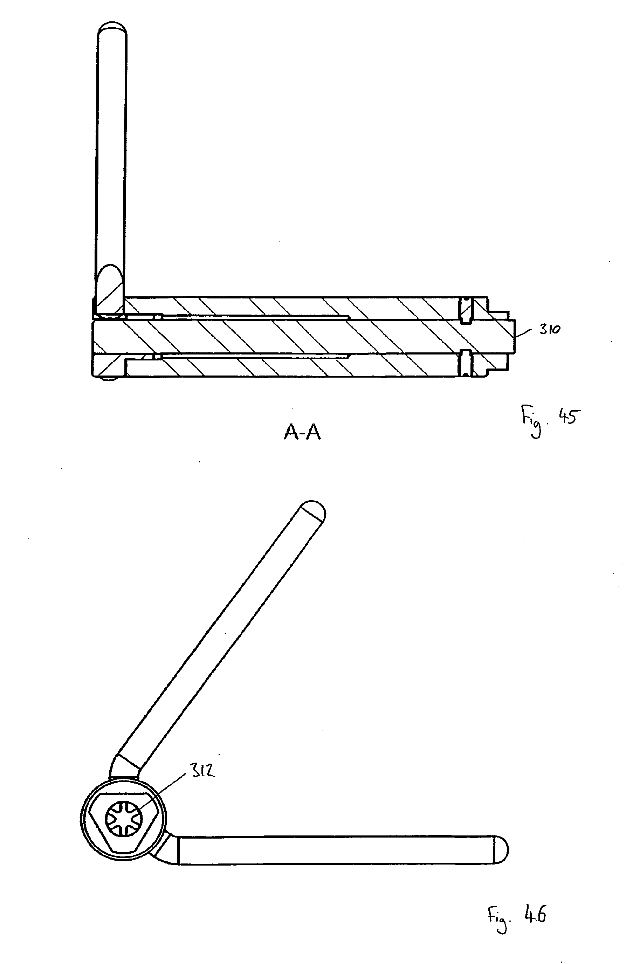

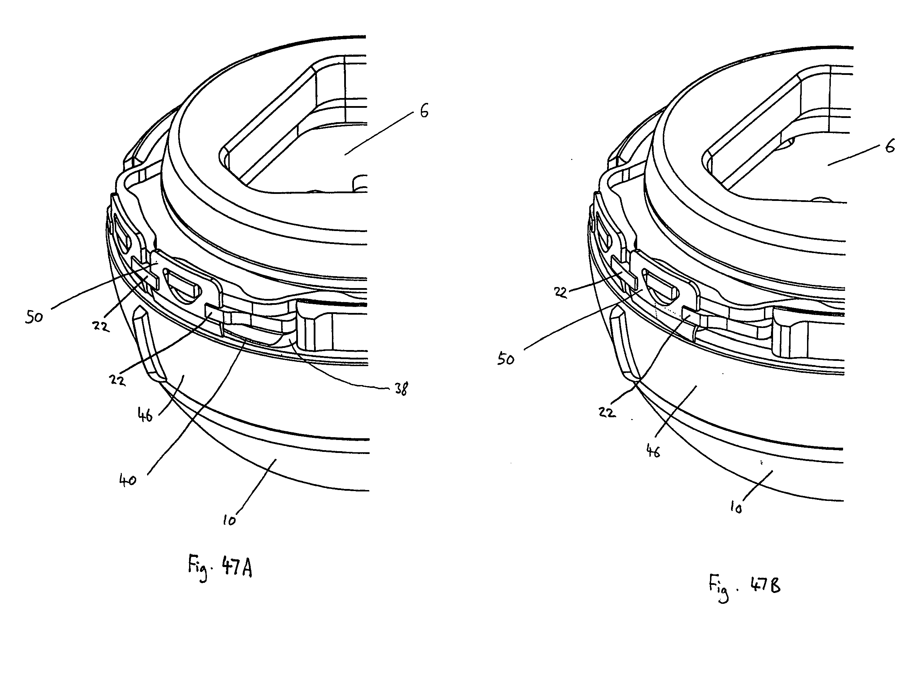

An embodiment of a surgical instrument 300, which can be used to activate the tab separation members described above and provide relative rotation of the tab separation member to the instrument head will now be described with reference to Figures 43-46. Figure 43 depicts a perspective view of the surgical instrument 300, Figure 44 a top view, Figure 45 a cross-section and Figure 46 a bottom view.

The surgical instrument comprises a first, generally tubular member 302 extending from a first engaging head 304 which has a formation of a truncated triangle, to engage the instrument head described above, with reference to the first and third embodiments. A rounded triangle engaging head (not shown) may be used with the second embodiment. A handle 306 extends in a radial direction from the first member 302.

A second member 308 is generally cylindrical and is provided within the tubular member 302, so that it is coaxial with member 302. A second engaging head 310 extends from the second member 308 and has a generally star shaped formation for engaging a corresponding formation provided on the tab separation member of the first and third embodiments. When used with the second embodiment, the second engaging head may have a generally triangular configuration (not shown). A second handle 314 extends from the second member 308.

In all the assemblies described above, rotation of the tab separation member through 360° is not required. The instrument 300 therefore limits the rotational position of the second member 308 relative to the first member 302. This is provided by locating the second handle 314 within a recess formed in the first member 300 which defines an arc of a circle. In this embodiment the arc is approximately 120°. This construction also allows handle 314 to be at the same axial position as handle 306, enhancing the ease of use.

In use, the first formation 304 is engaged with the instrument head and the second formation 312 is engaged with the tab separation member. Handle 306 is then held stationery while handle 314 is rotated relative to handle 306. This rotates formation 312 relative to formation 304, rotating the tab separation member relative to the instrument head so that the cam surface engages the weak region of the tabs and breaks the tabs from the circumferential band of the attachment member.

Although various embodiments have been described, it will be appreciated that the features of the above-described embodiments can be combined in other permutations than specifically as described. For example, different forms of tab separation members may be used in any of the embodiments. Various other modifications will suggest themselves to the person skilled in the art and are included within the scope of the invention, which is defined by the appended claims.

## Claims

1. An assembly comprising:
a medical implant comprising:
a cup prosthesis (10) having a circumference and a central axis which defines an axial direction and a radial direction; and
an attachment member (8) applied around the circumference of the cup, wherein the attachment member comprises:
a circumferential band (46); and
at least two tabs (50) extending in the axial direction from the circumferential band and having a circumferential region of weakness (60), each of the at least two tabs delimiting an opening; and
an instrument head (2, 24, 102, 202) attached to the attachment member and engaged with the cup prosthesis, the instrument head comprising an axial retaining element extending from the instrument head and engaged with the opening of the at least two tabs, **characterised in that** the axial retaining element (12, 112, 212) extends from the instrument head in the radial direction and each of the at least two tabs delimit an opening (54) in the radial direction adapted for engagement by the radially extending axial retaining element.

2. An assembly according to claim 1, wherein each of the at least two tabs (50) delimits an opening (54) which has a substantially flat portion (56) parallel to the circumferential band (46) and a tapering portion (58) which tapers in the direction towards the circumferential band.

3. An assembly according to claim 2, wherein each of the at least two tabs (50) delimit an opening (54) which is generally triangular.

4. An assembly according to claim 3, wherein each of the at least two tabs (50) delimits an opening (54) which is generally triangular and has two sides of equal length that define the tapering portion.

5. An assembly according to any one of claims 2 to 4, wherein the tapering portion tapers to a rounded apex (58).

6. An assembly according to any of claims 1 to 5, wherein the attachment member comprises at least three tabs (50).

7. An assembly according to any of claims 1 to 6, wherein the tabs (50) are equi-angularly spaced around the circumference of the circumferential band (46).

8. An assembly according to any preceding claim, wherein a plurality of tabs (50) are provided at a plurality of different positions around the circumference of the circumferential band (46).

9. An assembly according to any one of the preceding claims, wherein the circumferential band (46) has a height in the axial direction and each of the at least two tabs (50) extend in the axial direction for a distance which is less than or equal to the height of the circumferential band.

10. An assembly according to any preceding claim, wherein the circumferential band (46) includes one or more axial ribs (48), the or each axial rib protruding from the circumferential band in the radial direction and extending in the axial direction.

11. An assembly according to claim 1, wherein the axial retaining element (12, 112) is formed from a single piece with at least two radial extensions (44, 144) to engage respective openings in the at least two tabs.

12. An assembly according to claim 1, wherein the axial retaining element comprises a plurality of discrete members (212), each of the discrete members engaging at least one of the openings.

13. An assembly according to any preceding claim, wherein the axial retaining element (12, 112) is moveable in a radial direction.

14. An assembly according to claim 13, wherein the axial retaining element (12, 112) is biased in an outward radial direction.

## Patentansprüche

1. Baugruppe, umfassend:
ein medizinisches Implantat, umfassend:
eine Schalenprothese (10) mit einem Umfang und einer Mittelachse, welche eine axiale Richtung und eine radiale Richtung festlegt; und
ein Befestigungsglied (8), welches um den Umfang der Schale herum angebracht ist, wobei das Befestigungsglied umfasst:
ein Umfangsband (46); und
zumindest zwei Ösen (50), welche sich in die axiale Richtung von dem Umfangsband erstrecken und eine Umfangsschwachstellenregion (60) aufweisen, wobei jede der zumindest zwei Ösen eine Öffnung abgrenzt; und
einen Instrumentenkopf (2, 24, 102, 202), welcher an dem Befestigungsglied befestigt ist und mit der Schalenprothese ineinandergreift, wobei der Instrumentenkopf ein axiales Rückhalteelement umfasst, welches sich von dem Instrumentenkopf erstreckt und mit der Öffnung der zumindest zwei Ösen ineinandergreift, **dadurch gekennzeichnet, dass** das axiale Rückhalteelement (12, 112, 212) sich von dem Instrumentenkopf in die radiale Richtung erstreckt und jede der zumindest zwei Ösen eine Öffnung (54) in der radialen Richtung abgrenzt, ausgelegt zum Ineinandergreifen durch das sich radial erstreckende Rückhalteelement.

2. Baugruppe gemäß Anspruch 1, wobei jede der zumindest zwei Ösen (50) eine Öffnung (54) abgrenzt, welche einen im Wesentlichen flachen Anteil parallel zu dem Umfangsband und einen sich verjüngenden Anteil (58), welcher sich in die Richtung hin zu dem Umfangsband verjüngt, aufweist.

3. Baugruppe gemäß Anspruch 2, wobei jede der zumindest zwei Ösen (50) eine Öffnung (54) abgrenzt, welche im Allgemeinen dreieckig ist.

4. Baugruppe gemäß Anspruch 3, wobei jede der zumindest zwei Ösen (50) eine Öffnung (54) abgrenzt, welche im Allgemeinen dreieckig ist und zwei Seiten von gleicher Länge aufweist, welche den sich verjüngenden Anteil festlegen.

5. Baugruppe gemäß einem der Ansprüche 2 bis 4, wobei der sich verjüngende Anteil sich zu einem abgerundeten Scheitelpunkt (58) verjüngt.

6. Baugruppe gemäß einem der Ansprüche 1 bis 5, wobei das Befestigungsglied zumindest drei Ösen (50) umfasst.

7. Baugruppe gemäß einem der Ansprüche 1 bis 6, wobei die Ösen (50) gleichwinklig um den Umfang des Umfangsbands (46) herum beabstandet sind.

8. Baugruppe gemäß einem der vorhergehenden Ansprüche, wobei eine Mehrzahl von Ösen (50) an einer Mehrzahl von verschiedenen Positionen um den Umfang des Umfangsbands (46) herum bereitgestellt sind.

9. Baugruppe gemäß einem der vorhergehenden Ansprüche, wobei das Umfangsband (46) eine Höhe in der axialen Richtung aufweist und jede der zumindest zwei Ösen (50) sich in die axiale Richtung für eine Distanz erstreckt, welche geringer als die oder gleich der Höhe des Umfangsbands ist.

10. Baugruppe gemäß einem der vorhergehenden Ansprüche, wobei das Umfangsband (46) eine oder mehrere axiale Rippen (48) beinhaltet, wobei die oder jede axiale Rippe von dem Umfangsband in der radialen Richtung hervorsteht und sich in die axiale Richtung erstreckt.

11. Baugruppe gemäß Anspruch 1, wobei das axiale Rückhalteelement (12, 112) aus einem einzigen Stück bzw. einstückig mit zumindest zwei radialen Erweiterungen (44, 144) geformt ist, um mit den entsprechenden Öffnungen in den zumindest zwei Ösen ineinanderzugreifen.

12. Baugruppe gemäß Anspruch 1, wobei das axiale Rückhalteelement eine Mehrzahl von separaten bzw. diskreten Gliedern (212) umfasst, wobei jedes der separaten bzw. diskreten Glieder mit zumindest einer der Öffnungen ineinandergreift.

13. Baugruppe gemäß einem der vorhergehenden Ansprüche, wobei das axiale Rückhalteelement (12, 112) in einer radialen Richtung beweglich ist.

14. Baugruppe gemäß Anspruch 13, wobei das axiale Rückhaltelement (12, 112) in einer nach außen gerichteten radialen Richtung vorgespannt ist.

## Revendications

1. Ensemble comprenant :
un implant médical comprenant :
une prothèse en forme de cupule ayant une circonférence et un axe central qui définit une direction axiale et une direction radiale ; et
un élément de fixation (8) appliqué autour de la circonférence de la cupule, l'élément de fixation comprenant :
une bande circonférentielle (46) ; et
au moins deux pattes (50) s'étendant dans la direction axiale depuis la bande circonférentielle et présentant une région de faiblesse circonférentielle (60), chacune des au moins deux pattes délimitant une ouverture ; et
une tête d'instrument (2, 24, 102, 202) fixée à l'élément de fixation et en prise avec la prothèse en forme de cupule, la tête d'instrument comprenant un élément de retenue axial s'étendant depuis la tête d'instrument et en prise avec l'ouverture des au moins deux pattes, **caractérisé en ce que** l'élément de retenue axial (12, 112, 212) s'étend depuis la tête d'instrument dans la direction radiale et chacune des au moins deux pattes délimite une ouverture (54) dans la direction radiale adaptée pour l'entrée en prise par l'élément de retenue axial s'étendant radialement.

2. Ensemble selon la revendication 1, dans lequel chacune des au moins deux pattes (50) délimite une ouverture (54) qui présente une partie sensiblement plate (56) parallèle à la bande circonférentielle (46) et une partie de rétrécissement (58) qui rétrécit dans la direction vers la bande circonférentielle.

3. Ensemble selon la revendication 2, dans lequel chacune des au moins deux pattes (50) délimite une ouverture (54) qui est globalement triangulaire.

4. Ensemble selon la revendication 3, dans lequel chacune des au moins deux pattes (50) délimite une ouverture (54) qui est globalement triangulaire et possède deux côtés de longueur égale qui définissent la partie de rétrécissement.

5. Ensemble selon l'une quelconque des revendications 2 à 4, dans lequel la partie de rétrécissement rétrécit vers un sommet arrondi (58).

6. Ensemble selon l'une quelconque des revendications 1 à 5, dans lequel l'élément de fixation comprend au moins trois pattes (50).

7. Ensemble selon l'une quelconque des revendications 1 à 6, dans lequel les pattes (50) sont espacées de façon équiangulaire autour de la circonférence de la bande circonférentielle (46).

8. Ensemble selon l'une quelconque des revendications précédentes, dans lequel une pluralité de pattes (50) sont disposées dans une pluralité de positions différentes autour de la circonférence de la bande circonférentielle (46).

9. Ensemble selon l'une quelconque des revendications précédentes, dans lequel la bande circonférentielle (46) a une hauteur dans la direction axiale et chacune des au moins deux pattes (50) s'étend dans la direction axiale sur une distance qui est inférieure ou égale à la hauteur de la bande circonférentielle.

10. Ensemble selon l'une quelconque des revendications précédentes, dans lequel la bande circonférentielle (46) comprend une ou plusieurs nervures axiales (48), et la ou chaque nervure axiale faisant saillie depuis la bande circonférentielle dans la direction radiale et s'étendant dans la direction axiale.

11. Ensemble selon la revendication 1, dans lequel l'élément de retenue axial (12, 112) est formé d'un seul tenant avec au moins deux extensions radiales (44, 144) pour entrer en prise avec des ouvertures respectives dans les au moins deux pattes.

12. Ensemble selon la revendication 1, dans lequel l'élément de retenue axial comprend une pluralité d'éléments distincts (212), chacun des éléments distincts entrant en prise avec au moins une des ouvertures.

13. Ensemble selon l'une quelconque des revendications précédentes, dans lequel l'élément de retenue axial (12, 112) peut bouger dans la direction radiale.

14. Ensemble selon la revendication 13, dans lequel l'élément de retenue axial (12, 112) est sollicité dans une direction radiale vers l'extérieur.
